# EUROPEAN PATENT SPECIFICATION

(11) **EP 3 419 557 B1**
(45) Date of publication and mention of the grant of the patent: **08.06.2022**
(21) Application number: 17714508.3
(22) Date of filing: 24.02.2017
(51) Int. Cl.: A61D 17/00, A01J 5/04

(54) **MILK ANALYSER SYSTEM AND METHOD**
SYSTEM UND VERFAHREN FÜR MILCHANALYSE
SYSTÈME D'ANALYSEUR DE LAIT ET PROCÉDÉ

(30) Priority: 26.02.2016 GB 201603391
(43) Date of publication of application: 02.01.2019
(73) Proprietor: Milkalyser Limited, Exmouth, Devon EX8 1RL (GB)
(72) Inventor: MOTTRAM, Toby T F, Whimple Devon EX5 2TD (GB)
(74) Representative: Octrooibureau Van der Lely N.V.
(86) International application number: PCT/GB2017/050502
(87) International publication number: WO 2017/144913

(56) References cited:
- US-A- 5 208 147
- US-A- 5 743 209
- US-A1- 2010 285 523

## Description

### FIELD OF THE INVENTION

This invention relates to the field of milk analysis, in particular a milk analyser system for detecting the presence of a biomarker, and a method of using the system.

### BACKGROUND

Milk carries biomarkers that can be indicators of animal health or fertility. Measuring the concentration of these biomarkers and relaying the information via the internet is an increasing requirement for modern precision management systems. A specific example of the need for new inventions is in the measurement of progesterone in milk.

A preferred embodiment of the milk analyser system measures progesterone in milk in a milking parlour. The concentration of progesterone in milk can be used as a means of detecting the onset of ovulation, infertility and pregnancy in the cow from whom the milk is extracted. The invention will make the automated measurement of progesterone in milk a low cost and routine process.

The calving index or numbers of days between calving has been steadily rising since the introduction of artificial insemination at about 1-2 days per year and in 2009 it was 420 days in the UK. A number of studies in 1992 showed 95% of cows had active ovaries. The greatest influence on the cow fertility is management, with 32% of cycles detectable by progesterone not being recognised by the herdsman. Between 5% and 21% of cows were inseminated at the incorrect time in the cycle.

The sequence of events associated with the ovarian cycle in dairy cows is well known and described in a number of standard texts. For the purposes of detection of the stage of the ovarian cycle the cow may emit a number of hormonal and behavioural signals which may be measured externally. Progesterone levels are elevated from 4-16 days post ovulation then fall to a nadir. The hormone oestradiol has an inverse relationship to progesterone so that as progesterone levels fall that of oestradiol rises. The oestradiol stimulates the oestrus behaviour which is most commonly used to select cows for insemination. The optimum time to inseminate the cow is 6-12 hours after the peak in oestradiol concentration. There is a diurnal peak of oestrus behaviour early in the morning and late at night. Standing to be mounted is not a reliable indicator of a cow being suitable to be served, as many as 21% of ridden cows are pregnant. A 1984 study surveyed 35000 cows in 255 herds and showed that 26% of interservice intervals were greater than 48 days and that 19% of cows were served at an interval of 1-17 and 25-35 days suggesting that one or other of the observed oestrus events was not accompanied by ovulation whose periodicity has been established as very consistent. Frequently the performance of a device for insemination is compared with the ability of the herdsman to observe behavioural indications of oestrus. A more satisfactory reference to use is milk or blood progesterone level, which gives a reliable indication of ovulation.

A preferred embodiment of the present invention is a device to measure progesterone in milk to detect ovulation, infertility and pregnancy. However, the system and technique is extensible to other assays such as for mastitis, infectious disease and concentrations of ketones in milk.

The current most common form of oestrus detection and by proxy the ovulation cycle is the use of collars or pedometers to measure the rise of activity when cows are in oestrus. Collars for detecting oestrus have been available since the early eighties. The early versions used simple mercury tilt switches or rolling balls to count the number of head movements. These were relatively expensive devices until the integrated circuits with tri-axial accelerometers became available in the 1990s. Since the advent of cheap tri-axial accelerometers and digital signal processor chips the collar has been shown as a tool that can not only detect oestrus but also lying behaviour, lameness, location, and with the collection of audio data rumination and eating activities. These collars have been used widely and models are slowly emerging as to how the data can be integrated into a wholistic management system. Commercial activity has probably been more advanced than research reports.

Publication WO2006077589 describes a method and device for detecting oestrus in animal by sensing along time the motion of the animal and identifying when the sensed motion is not related to eating periods of the animal. Use of the said device has achieved 76.9% sensitivity (SN), 99.4% Specificity (SP) and 82.4% positive predictive value (PPV) of ovulation detection. While activity only collars achieved 62.4% SN, 99.3 SP, 76.6% PPV all in comparison to progesterone analysis of milk as the gold standard. There have been many attempts at improvements on the basic concept but none are as accurate as progesterone analysis. The disadvantages of collars are that they need to be fitted to each animal to be monitored unlike a parlour based system which just gets used more as cow numbers rise. They are subject to major wear and tear and as the cows change weight through the year the fit of the collar changes and thus the movements are recorded differently and algorithms need to adjust.

Experiments to measure the efficacy of oestrus detection systems use progesterone assay as the standard calibration tool. A sample of milk is taken and analysed once per day firstly to identify that oestrus cycling has begun and then to identify the drop in progesterone which precedes ovulation by approximately 48 hours. The principle of these tests is that an antibody to progesterone is attached to a plastic surface during manufacture. The farmer or veterinarian then adds milk. Progesterone in the milk then attaches to the antibody coated on the surface. A reagent is added and rinsed out and a colour change is observed to identify the amount of progesterone present in the milk.

Although in laboratory terms this is a simple test it does require a small amount of skill, some space and correct timing to get consistent results. Analysis of progesterone levels in milk can not only be used to monitor the stage of the oestrous cycle but also to detect pregnancy and to identify early ovarian disorders. Taking and analysing milk samples away from the milking parlour is labour intensive, strategies have been needed to minimise the number of samples needed to improve fertility. Experimental work during the 1980s established a sampling protocol and showed that inseminating cows on the basis of progesterone profiles using laboratory analysis of samples could achieve significant improvements in fertility. It has been shown that 99% of 88 ovulations were correctly identified using on-farm progesterone kits compared with 78% in a control group monitored conventionally. Milk samples were taken three times a week starting 25 d. post partum. Once an ovulation had been detected by a fall in progesterone concentration to below 4 ng/ml and a subsequent rise to more than 7 ng/ml sampling was suspended for 15 days. Sampling resumed on alternate days until a fall in progesterone indicated the onset of oestrus. The cows were then inseminated 48 hours following the fall in progesterone. Sampling continued so as to determine whether oestrus had been correctly identified or whether the cow had conceived.

With regard to detection of ovarian malfunction, a study of over 500 cows in a controlled trial suggested that alternate day progesterone profiles were a better method of analysing ovarian malfunction than rectal palpation. Studies show little incidence of ovarian dysfunction and that the principal cause of extended calving intervals was a failure to detect oestrus. Cows were diagnosed as anoestrus when progesterone was below 4 ng/ml for 30 d post partum. Ovulation was deemed to have occurred if progesterone was below 4 ng/ml followed by 5 days of progesterone rising above 4 ng/ml, with at least one sample greater than 7 ng/ml.

Normal cycling was detected by an increase of progesterone which remained high for more than 5 days and less than 18 days. Insemination was assumed to be correctly timed if progesterone increased in the 2-6 days following. Conception was identified if ovulation and correctly timed insemination coincided and that progesterone remained above 4 ng/ml for more than 20 days. If progesterone was greater than 4 ng/ml for more than 30 days after conception then pregnancy was assumed to be established. The progesterone diagnosis proved to be more accurate than the rectal palpation and indicated that 36.5 % of clinical diagnoses were incorrect. At 42 days post partum 92 % of cows were cycling normally which reinforces the view that identification of the oestrus cycle is the problem. Ovulation detection rates of 98% using progesterone assay are routine and one embodiment of this invention will make them simple and automated.

A preferred embodiment of the invention uses a biosensor. The term biosensor is loosely used to describe a number of different devices, the objectives of which are to identify specific complex biological molecules by a change in electrical or opto-electronic signal. A biosensor system consists of a sensor, a system to interrogate it and a microcomputer to convert the electrical signal into a format to be displayed to an operator or computer program. The sensing methods are usually based on a monoclonal antibody which is specific to the compound being detected.

It has been shown that realistic levels of progesterone would only produce a change in mass of 0.4% on a Quartz Crystal Microbalance and were very close to the noise level of the microelectronics so they proposed an alternative method by automating an ELISA test for on-line measurement of progesterone in bovine milk and detection of oestrus. The biosensor used an enzyme immunoassay format for molecular recognition, which was developed to run in approximately eight minutes. The sensor was designed to operate on-line in a dairy parlour using microinjection pumps and valves for fluid transport, fibre optics and photodiodes for light measurement, and a control computer for sequencing. Calibration showed a dynamic response between 0.1 and 5ng/ml progesterone in milk.

A reported device was based on a disposable screen-printed amperometric progesterone biosensor, operated in a competitive immunoassay. The biosensor comprised a monoclonal anti-progesterone antibody (mAb) immobilised on the working area of a screen-printed carbon electrode (SPCE). It relied upon a reduction in the binding of alkaline phosphatase-labelled progesterone in the presence of endogenous milk progesterone. The enzyme substrate was naphthyl phosphate and the 1-naphthol generated in the enzymatic reaction was electrochemically oxidised, producing a signal inversely proportional to the concentration of unlabelled progesterone in milk. This SPCE-based immunosensor for progesterone was incorporated into a thin-layer flow cell offering advantages such as on-line analysis and improved fluid handling with the possibility of future automation. Also demonstrated was a system based on applying a milk sample to an electrochemical biosensor and reading the electrical response.

Also developed was the Herd Navigator system, which combines automated sampling and five sensing systems including progesterone in milk. It was reported that heat detection rates of 95%- 97% were being achieved on three farms in Denmark with the number of days open reduced by 20 days in the first year of operation. Pregnancy rates also increased significantly in a very short time: up to 50%. Herd Navigator automatically measures the level of progesterone in milk, software indicates insemination time, lists animals for final pregnancy confirmation, indicates early abortion and lists the cows with risk for cysts and prolonged anoestrus. Also reported were heat detection rates of two farms in the Netherlands using Herd Navigator as 94 & 99%. Pregnancy rates of 42 & 46% were reported. It is believed that a number of Herd Navigator farmers have stopped performing manual pregnancy tests and saved between €250 and €350 per cow per year. Herd Navigator consists of a large analyser boxed with controlled environment and complex plumbing system to bring the milk samples to the analyser from the short milk tubes. It is designed to fit into parlours with a small number of milking points when newly installed and would be difficult to retrofit into existing parlours, particularly large systems that are working continuously.

In the modern dairy milking systems are highly automated to ensure a high number of cows can be milked per hour. Any new system of automatically measuring biomarkers in milk must link to the existing parlour automation to know which cow is to be analysed in which milking position. Most milking parlours operate for many hours a day throughout the year and so time to install analysers and plumbing and vacuum and other services pipes, lines, wires and other connections must be minimised or removed. It would be beneficial to provide a system that could be installed in minutes and with the option of using wireless to link to the milking system. A preferred embodiment of the invention provides an automated ovulation, infertility and pregnancy detection system for dairy animals, that requires no external wiring or plumbing. It can be fitted in minutes by attaching the system box in a level position to a rigid surface adjacent to the cow, cutting the long milk tube and connecting the cut ends to the entry and exit pipes.

A preferred embodiment of the invention is a fluid analyser suitable for installation in any milking system conforming to ISO 5570. The embodiment combines a number of features that provide an automated sampler/analyser for direct retrofitting to existing milking parlours.

US 5208147 describes a means for measuring a characteristic in a sample fluid, and is regarded as the closest prior art to the system of claim 1. Preferred embodiments are described in the dependent claims.

US 2010/0285523 describes a system and method for analysing fluids. US 5743209 describes a system and method for monitoring and controlling milk production at dairy farms.

### SUMMARY OF THE INVENTION

According to an aspect of the invention, there is provided a milk analyser system for detecting the presence of a biomarker, comprising: a colorimetric sensor which is arranged to change colour in the presence of a biomarker to be detected; and an optically transparent container which contains the sensor, the container being openable in use to allow milk received by the system to pass over the sensor in order to provide an indication of the biomarker in the milk, any change in colour in the sensor being detectable by viewing the sensor through the container, wherein the sensor container forms part of an array of sensor containers that can be driven through the system in use.

A colorimetric sensor, e.g. a colorimetric lateral flow immunosensor, needs to be packaged to preserve it during storage. In this invention the sensor packaging beneficially becomes part of the deployment and data capture system. That is, the packaging contains the sensor and can be opened to expose the sensor to the milk to be tested. Furthermore the opened packaging can act as a reservoir, holding a sample of milk by gravity against the sensor surface. Thus the packaging of the sensor is very efficient, serving both to preserve the sensor and also, when opened, to provide access to the sensor by the milk. Also the packaging is transparent and therefore permits a colour change of the sensor to be read, preferably automatically by an optical system. The invention therefore advantageously provides the automation of sensor monitoring of milk, preferably with respect to progesterone and thus cow fertility management.

The sensor containers may be provided on a movable cassette. The cassette may comprise a flexible web on which the sensor containers are formed.

The milk analyser system may comprise a cutter for opening each container, to allow the milk to pass over the respective sensor.

The milk analyser system may comprise a cam which is operable to deform and reform the each container in order to provide a partial vacuum in the opened container, thereby to draw the milk over the sensor.

The milk analyser system may comprise: a needle which is operable to puncture the each container; and a pump which is connected to the needle and operable to provide a partial vacuum in the opened container, thereby to draw the milk over the sensor

The each container may be configured to be prevacuumised, such that opening the container provides a partial vacuum in the container, thereby to draw the milk over the sensor.

The milk analyser system may comprise an optical detector for detecting changes in the colour of the sensor in use. The milk analyser system may comprise a processor configured to process an output of the optical detector, to provide at least one of an immediate indication of, and a record of, the level of the biomarker in the milk.

The milk analyser system may be arranged within a watertight container, with an inlet for receiving the milk and an outlet for passing the milk out of the system.

The milk analyser system may be configured to communicate with a remote processor for processing an output of the system and providing a report to an operator.

According to another aspect of the invention, there is provided a method of operating a milk analyser system as described herein above. The biomarker may be at least one of: progesterone in solution measured in nanograms per millilitre; oestradiol; Follicle Stimulating Hormone; a ketone; urea; acute phase protein; somatic cell; and an antibody to an infectious disease agent. The infectious disease may be TB, brucellosis, or Bovine Viral Diarrhoea.

### BRIEF DESCRIPTION OF THE DRAWINGS

An example of the present invention will now be described with reference to the accompanying drawings, in which:
Figure 1 is a schematic drawing showing an example analyser system according to the invention;
Figure 2 shows a sampling system, which forms part of the analyser system of Figure 1;
Figure 3 shows sensor and blister packages of the sampling system; and
Figure 4 shows an alternative arrangement of the sampling system.

### DETAILED DESCRIPTION OF EMBODIMENTS OF THE INVENTION

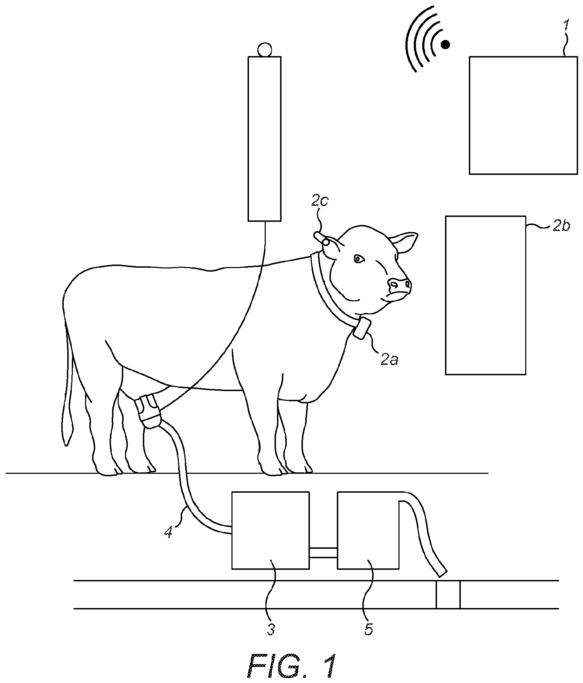

Referring to Figure 1, a system controller 1 is connected to the internet and communicates messages to a multiplicity of "milkalysers" and other devices. The system controller 1 is preferably a computer with communications capability and either contains or has access to a database containing status information about each cow to be milked. In order to identify a cow and sample and analyse milk from that cow, there is provided a radio frequency identification system, using either a transmitter 2a mounted on a collar or a transmitter mounted in an ear tag 2c, which can be activated and read by an antenna 2b. Alternatively the cow identity may be provided by a human operator entering the cow identity manually. The cow identity number can be transmitted to the system controller 1 which then looks up in the database whether the cow needs to be sampled and analysed at this time.

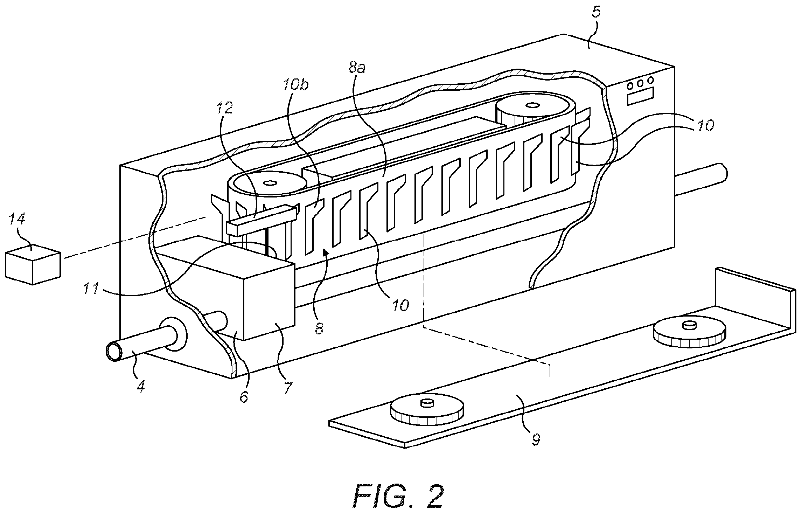

Referring also to Figure 2, a waterproof box contains a fluid analysis or sampling system 3 and is activated when it receives a signal to do so from the system controller 1.

There is also shown a sampler fitting in a long milk tube 4. The sampler when not activated acts as part of the long milk tube. Alternatively the sampler and analyser may be integrated into a milk metering device 5.

In some embodiments the milk analyser will be adjacent or attached to a milk metering device 5, so that milk flows through the sampler to the milk collection system in a conventional way as described in ISO 5570.

A sensor 6 is arranged to detect the start of milking. The sensor 6 may be integrated or connected from the milking system control to indicate when milk has started flowing through the sampler. A number of different sensors can be used to fulfil this function to detect one or more of milk flow, vacuum change, to activate the sampling system. As the release of milk varies from cow to cow and the concentration of progesterone changes during the milking process it is important that sampling is conducted at the same or approximately the same time during the milking sequence, this can in some embodiments be tuned by software but the default time will be typically 60 seconds from the application of vacuum to the long milk tube 4.

In use, a sampling pump 7 draws milk from the long milk tube 4. As the long milk tube 4 is under partial vacuum it is necessary to apply a stronger vacuum or use a method such as those disclosed in prior art. The milk sample is drawn into a container or well where it is under atmospheric pressure from which it can be drawn into sensor packages 10 (described in more detail herein below). The sampler returns unused milk to the milk line. During the wash sequence of the milking machine, the sampling pump operates to flush away milk residues and maintain a hygienic system.

Still referring to Figure 2, a reusable cassette 8 comprises a plurality of sensor packages 10. Alternatively the cassette 8 comprises just a single sensor package 10. In this embodiment the cassette 8 comprises a battery for powering the electrical features of the system. Alternatively the battery can be provided separately from the cassette 8.

A carriage and mechanism 9 is arranged to advance the sensor packages 10 held in the cassette 8 to a new position. The mechanism 9 comprises a waterproof door to allow the cassette 8 to be secured and protected from washing and other activities of the milking parlour. The mechanism 9 includes a controllable motor to advance the sensor packages 10 into any indexed position. The mechanism 9 may be mounted in any position to advance the sensor packages 10 into position and avoid any liquids leaking from the rest of the system.

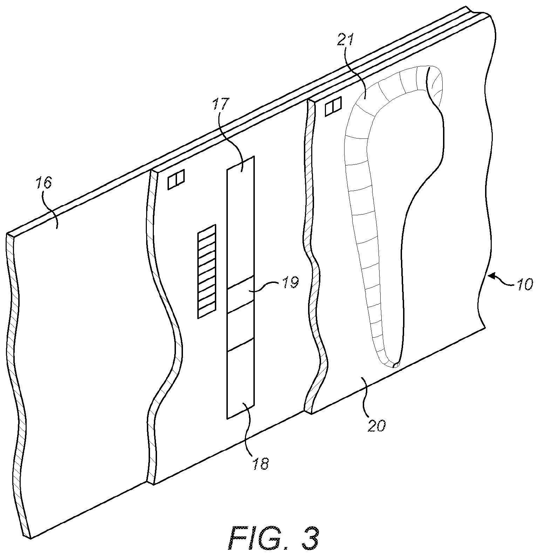

The cassette 8 comprises a flexible web 8a on which the sensor packages 10 are mounted or otherwise provided. Referring now also to Figure 3, each of the sensor packages 10 comprises a blister package 10a which includes a deformable bubble 10b that can be depressed and released to control the volume of milk applied to the sensor package 10. The surface of each blister package 10a is translucent to allow the colorimetric surface of the sensor to be visible to a camera. The blister package 10a also contains chemicals such as drying agents, or others, to keep the sensor in an active condition as long as possible. Each of the sensor packages 10 comprises at least one immunosensor 19 for measuring (different) analytes in the sample.

Referring now in particular to Figure 3, the sensor package 10 is made by adhering or otherwise depositing multiple layers of different material onto a flexible web. A tear-resistant base layer 16 of the blister package 10a is made waterproof, before adding a sensor layer 17 which comprises an absorbent end 18. A zone or section of the immunosensor 19 is arranged to change colour in a predictable manner after the liquid to be sampled has reacted with a substrate. A colour chart may be placed at a suitable location on the cassette 8 in order to permit the camera to capture a registration image for the individual cassette which may be subject to colour variation in the manufacturing process.

A knife 11 is arranged to pierce the base of the sensor package 10 to permit liquid to flow into the sensor package 10. As the cassette 8 rotates it activates the knife 11 so as to permit a controlled volume of sample fluid to be drawn onto the immunosensor 19.

When the sensor package 10 is advanced, a cam 12 (or other suitable sample-triggering means) depresses the bubble 10b of the blister package 10a, thereby blowing air or other packaging gas through the vent created by the knife 11 and creating a partial vacuum that draws a controlled volume of the sample onto the immunosensor 19.

A means of actuating the sample of milk into the sensor package 10 is disclosed as the movement of the sensor package 10 past the cam 12, releasing the blister package 10a to reform itself, creating a partial vacuum in the package and thereby drawing a sample into the sensor package 10. Alternatively a vacuum may be applied through a needle puncturing a septum in the sensor package 10 and applying a vacuum directly, for example via a pump. The result of this operation is to draw the sample into the package where it activates the lateral flow immunosensor 19.

The blister package 10a may alternatively be prevacuumised to enable the piercing and the milk sampling to be a simultaneous operation. In this case the cam 12 and the bubble 10b of the blister package 10a are unnecessary.

It will be understood by the skilled reader that the knife 11 is just one possible means of providing the liquid with access to the immunosensor 19, and other means are contemplated. For example, some suitable element, including but not limited to a needle, a probe, or a saw, may be provided to perforate, puncture, bore, cut, slice, slash, break, fracture, penetrate, or otherwise open the sensor package 10. All of these and other suitable means are considered to be within the scope of the claimed invention, provided that they somehow open the sensor package 10 so that the fluid can reach the immunosensor 19.

A combined (or alternatively separate) camera and microprocessor with light source 14 is provided to capture and process the image of the colour changing section of the immunosensor 19. The data may comprise one or more of date, time, cow number, colour change, a full image, the number of the sensor, the temperature, and any other data that may affect the quality of analysis.

The microcontroller 14 in this embodiment comprises software to receive a signal from the system controller 1 and then to perform a sequence of operations to advance the immunosensor 19 into a sampling position, wait until sample has propagated through the immunosensor 19 (this may be a number of minutes) and then capture the colour change on the immunosensor 19 and capture any other metadata before signalling the end of the analysis cycle. During the wash phase of the milking machine the microcontroller 14 activates the sampling pump to ensure that the system is fully cleaned.

The microcontroller 14 may calculate the stage of the fertility cycle, or the analysis may be performed by the system controller 1 or elsewhere by communication. The information may be used immediately to inform the person milking the cows that an animal needs attention or diversion for breeding. In some embodiments the method of returning information to the herdsman may be from a desktop or smartphone display of information. The algorithm by which a cow is deemed ready to ovulate, or be infertile or pregnant, has been disclosed in the prior art.

The cassette 8 is a removable item that is ejected by a button or other method of activation. The cassette 8 will appropriately be ejected when the sensor packages 10 have all been used or become out of date. In another embodiment the fluid analyser can be interlocked or provide warnings to the milking system, so that if analyses could not be performed the milking system could shut down. The eject button for the cassette 8 may activate a mechanism which only releases the cassette 8 when the computer has fully shut down.

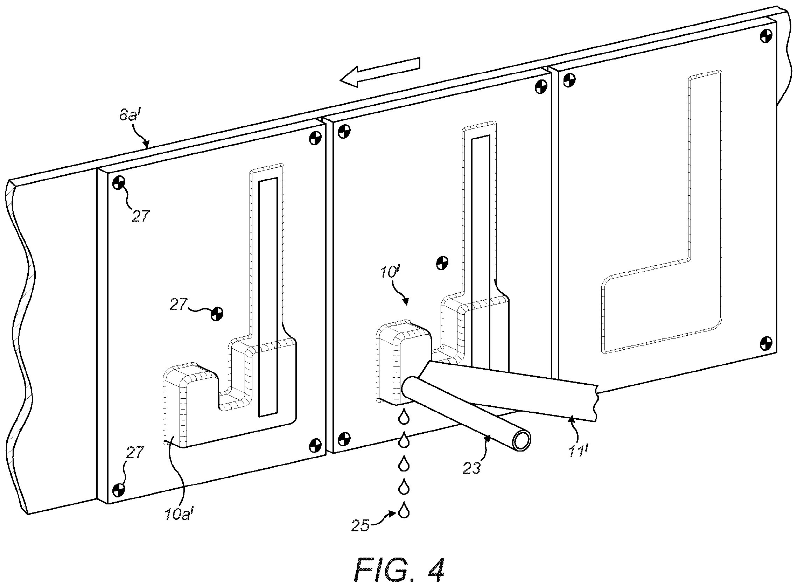

Referring now to Figure 4, there is shown a part of the flexible web 8a' comprising three blister packages 10a', the left-hand package being intact, the right-hand package being already used, and the central package presently being cut open. In this embodiment a pair of knives 11' (or alternatively a single knife) is arranged to cut a slot in the blister package 10a'. A filler pipe 23 is provided by which the milk sample can be injected into the deformed package. The cam, for depressing the blister package 10a', may be made integral with the filler pipe 23 or may instead be provided separately. The cam may also include an outlet from a pump, for injecting the sample fluid into a well at the base of the sensor package 10', so that the wicking effect of the sensor will draw the sample into the lateral flow matrix and enable the sensor to change signal. The blister package 10a', or instead a septum within the package, may alternatively be pierced by a hollow needle for the injection of the sample fluid into the sensor package 10'. Furthermore other means are envisaged for opening the sensor package 10, so that the fluid can reach the sensor, as has already been discussed herein above.

A means of disposing of surplus milk 25 within the deformed blister package 10a' may be provided, for example by cutting a drain with a separate knife to allow the package to drain, or by peeling the sensor package 10 from its backing strip or using a cam to crush any residual fluid to prevent contamination.

Optical or physical registration features 27 may be provided, to locate the sensor packages 10' in the correct position for the camera.

A preferred embodiment of the fluid analyser system can be fitted or retrofitted to any existing milking system and software that can be linked into existing herd management packages or be free standing. One or more analyser boxes are controlled by an internet linked system controller, typically controlling the multiplicity of analysers within a single milking parlour.

The operating principle of the sampler/analyser is that a sample of milk is drawn in by vacuum into an aseptically packed sensor, or alternatively with a syringe pump operating directly from the milk line. The controlled volume of milk is applied to a lateral flow colorimetric immunoassay which over a period of time (e.g. minutes) changes its colour response. The colour change of the sensor is captured by a microcomputer camera system and can be analysed and transmitted to a central database.

Another embodiment would place a multiplicity of immunosensors 19 for different analytes within the same blister package 10a so that multiple assays could be conducted simultaneously.

The described sensor package(s) would be all that is necessary for a single analysis with an embodiment using a smartphone to capture the sensor image and if required transmit the result to a system controller embodied on the internet. This embodiment might be appropriate where individual animals were to be sampled, for example in hand milking or small scale operations.

A preferred embodiment comprises an enclosure to protect the driving mechanism and electronics from the operational environment. However, other embodiments would be appropriate to sense the fluid flow separately from the analyser box. The start of milking could be detected by using an automated cluster removal system switch, or by a button pressed by a milking operative.

In another embodiment the sampling pump 7 could be at a distance from the analyser box 3 with milk brought through a pipe. Such an embodiment would have the advantage of being more flexible and even permit a multiplicity of milking points to be serviced by a single analyser box.

A preferred embodiment uses a rechargeable or disposable battery to be part of the cassette driving mechanism 9 and this battery can be the sole power source for the computer, camera, lights, sampling pump, sensors and motor. The embodiment includes an interlock linked to the active mode of the computer so that the computer is shut down before the cassette 8 can be removed.

The sensor package 10 may be made by depositing multiple layers of appropriate materials and finally encapsulating the immunosensor 19 under a clear deformable protective layer. The individual sensor packages 10 can be detached and used individually. In a preferred embodiment the strip of blister packaged sensors is made into a continuous web fitted into a cassette 8.

The box containing the components of the system is waterproof and dark inside. To enable the camera to function a light source is provided which is switched on to illuminate the sensor. In one embodiment a diffuse light source is used. Alternatively to reduce power consumption a casing made of translucent material for the box will allow a diffuse light to illuminate the sensor. Where a light source is used a polarising filter can reduce the reflections from the sensor package in the image. Alternatively a non-shiny sensor package material can be used. A combination of these techniques will permit an image to be captured with a high colour definition with limited reflections that could distort the colour read by the optical sensor. It will be understood that any change in colour may be with respect to only a region or specific portion of the sensor, rather than the sensor as a whole.

In some instances the outside of the transparent sensor package may become contaminated with debris or other material, causing the camera's view of the sensor to be obscured. The sensor package(s) may therefore include a removable outer layer, for example a peel-off layer, which can be removed to give the camera a clearer view of the sensor. The removable layer may itself be transparent, or it may be opaque.

The cam, injection needle, microswitches, camera and lights, drain, and knife or knives are all static features of the encapsulation surrounding the cassette. The encapsulation thus becomes part of the machine, ensuring that the movement of the transparent deformable sensor pack causes it to be ruptured, to permit milk to be applied to the sensor in a controlled manner and for an image or colour reading to be captured by the optical sensing system. The same features, most usefully the cam, can also be used to squeeze the deformed sensor package to remove any residual milk, to prevent the growth of cheese or mould in the system until the cassette is removed.

It should be understood that the invention has been described in relation to its preferred embodiments and may be modified in many different ways without departing from the scope of the invention as defined by the accompanying claims.

For example, the cassette of sensors can be implemented by a single strip of sensors with two ends, or a continuous web. The cassette of sensors can be a service item where used sensors are replaced and new ones fitted.

## Claims

1. A milk analyser system for detecting the presence of a biomarker, comprising:
a colorimetric sensor (9) which is arranged to change colour in the presence of a biomarker to be detected; and
an optically transparent container (10) which contains the sensor (9), the container (10) being openable in use to allow milk received by the system to pass over the sensor (9) in order to provide an indication of the biomarker in the milk, any change in colour in the sensor (9) being detectable by viewing the sensor (9) through the container (10),
wherein the sensor (9) container (10) forms part of an array of sensor containers (10) that can be driven through the system in use.

2. A milk analyser system according to claim 1, wherein the sensor containers (10) are provided on a movable cassette (8).

3. A milk analyser system according to claim23, wherein the cassette (8) comprises a flexible web (8a) on which the sensor containers (10) are formed.

4. A milk analyser system according to claim 3, comprising a cutter (11) for opening each container (10), to allow the milk to pass over the respective sensor (9).

5. A milk analyser system according to claim 4, comprising a cam (12) which is operable to deform and reform the each container (10) in order to provide a partial vacuum in the opened container (10), thereby to draw the milk over the sensor (9).

6. A milk analyser system according to claim 4, comprising:
a needle which is operable to puncture the each container (10); and
a pump which is connected to the needle and operable to provide a partial vacuum in the opened container (10), thereby to draw the milk over the sensor (9).

7. A milk analyser system according to claim 4, wherein the each container (10) is configured to be prevacuumised, such that opening the container (10) provides a partial vacuum in the container (10), thereby to draw the milk over the sensor (9).

8. A milk analyser system according to any preceding claim, comprising an optical detector for detecting changes in the colour of the sensor (9) in use.

9. A milk analyser system according to claim 8, comprising a processor configured to process an output of the optical detector, to provide at least one of an immediate indication of, and a record of, the level of the biomarker in the milk.

10. A milk analyser system according to any preceding claim, wherein the system is arranged within a watertight container, with an inlet for receiving the milk and an outlet for passing the milk out of the system.

11. A milk analyser system according to any preceding claim, configured to communicate with a remote processor for processing an output of the system and providing a report to an operator.

12. A method of operating a milk analyser system according to any of the preceding claims.

13. A method according to claim 12, wherein the biomarker is at least one of:
progesterone in solution measured in nanograms per millilitre;
oestradiol;
Follicle Stimulating Hormone;
a ketone;
urea;
acute phase protein;
somatic cell; and
an antibody to an infectious disease agent.

14. A method according to claim 13, wherein the biomarker is an antibody to an infectious disease agent and the infectious disease is TB, brucellosis, or Bovine Viral Diarrhoea.

## Patentansprüche

1. Milchanalysesystem zum Nachweisen des Vorhandenseins eines Biomarkers, umfassend:
einen kolorimetrischen Sensor (9), der so ausgelegt ist, dass er bei Vorhandensein eines nachzuweisenden Biomarkers die Farbe ändert; und
einen optisch transparenten Behälter (10), der den Sensor (9) enthält, wobei der Behälter (10) bei Verwendung geöffnet werden kann, um zu ermöglichen, dass Milch, die durch das System empfangen wird, über den Sensor (9) fließt, um eine Anzeige des Biomarkers in der Milch bereitzustellen, wobei jegliche Farbänderung des Sensors (9) erfasst werden kann, indem der Sensor (9) durch den Behälter (10) beobachtet wird,
wobei der Behälter (10) des Sensors (9) zu einer Anordnung von Sensorbehältern (10) gehört, die bei Verwendung durch das System bewegt werden kann.

2. Milchanalysesystem nach Anspruch 1, wobei die Sensorbehälter (10) auf einer beweglichen Kassette (8) vorgesehen sind.

3. Milchanalysesystem nach Anspruch 23, wobei die Kassette (8) eine flexible Materialbahn (8a) umfasst, auf der die Sensorbehälter (10) ausgebildet sind.

4. Milchanalysesystem nach Anspruch 3, umfassend eine Schneideeinrichtung (11) zum Öffnen jedes Behälters (10), um zu ermöglichen, dass die Milch über den jeweiligen Sensor (9) fließt.

5. Milchanalysesystem nach Anspruch 4, umfassend einen Nocken (12), der zum Verformen und Umformen jedes Behälters (10) betätigt werden kann, um ein Teilvakuum im geöffneten Behälter (10) bereitzustellen, um dadurch die Milch über den Sensor (9) anzusaugen.

6. Milchanalysesystem nach Anspruch 4, umfassend:
eine Nadel, die zum Durchstechen jedes Behälter (10) betätigt werden kann; und
eine Pumpe, die mit der Nadel verbunden ist und zum Bereitstellen eines Teilvakuums im geöffneten Behälter (10) betätigt werden kann, um dadurch die Milch über den Sensor (9) anzusaugen.

7. Milchanalysesystem nach Anspruch 4, wobei jeder Behälter (10) konfiguriert ist, um prävakuumiert zu werden, derart, dass ein Öffnen des Behälters (10) ein Teilvakuum im Behälter (10) bereitstellt, um dadurch die Milch über den Sensor (9) anzusaugen.

8. Milchanalysesystem nach einem der vorhergehenden Ansprüche, umfassend einen optischen Detektor zum Erfassen von Farbänderungen des Sensors (9) bei Verwendung.

9. Milchanalysesystem nach Anspruch 8, umfassend einen Prozessor, der zum Verarbeiten einer Ausgabe des optischen Detektors konfiguriert ist, um mindestens eine von einer sofortigen Anzeige oder einer Aufzeichnung der Konzentration des Biomarkers in der Milch bereitzustellen.

10. Milchanalysesystem nach einem der vorhergehenden Ansprüche, wobei das System innerhalb eines wasserdichten Behälters mit einem Einlass zum Empfangen der Milch und einem Auslass zum Austreten der Milch aus dem System angeordnet ist.

11. Milchanalysesystem nach einem der vorhergehenden Ansprüche, konfiguriert zum Kommunizieren mit einem Remote-Prozessor zum Verarbeiten einer Ausgabe des Systems und Bereitstellen eines Berichts für eine Bedienperson.

12. Verfahren zum Betreiben eines Milchanalysesystems nach einem der vorhergehenden Ansprüche.

13. Verfahren nach Anspruch 12, wobei es sich bei dem Biomarker um mindestens eines von Folgenden handelt:
Progesteron in Lösung, gemessen in Nanogramm pro Milliliter;
Estradiol;
follikelstimulierendes Hormon;
ein Keton;
Harnstoff;
Akute-Phase-Protein;
somatische Zelle; und
einen Antikörper gegen einen Infektionskrankheitserreger.

14. Verfahren nach Anspruch 13, wobei der Biomarker ein Antikörper gegen einen Infektionskrankheitserreger ist, und die Infektionskrankheit TB, Bruzellose oder Bovine Virusdiarroe ist.

## Revendications

1. Système d'analyse de lait destiné à détecter la présence d'un biomarqueur, comprenant :
un capteur colorimétrique (9) qui est agencé pour changer de couleur en présence d'un biomarqueur devant être détecté ; et
un récipient optiquement transparent (10) qui contient le capteur (9), le récipient (10) pouvant être ouvert à l'usage pour permettre au lait reçu par le système de passer par-dessus le capteur (9) afin de fournir une indication du biomarqueur dans le lait, tout changement de couleur dans le capteur (9) étant détectable par observation du capteur (9) à travers le récipient (10),
dans lequel le récipient (10) du capteur (9) forme une partie d'un réseau de récipients à capteur (10) qui peuvent être entraînés à travers le système à l'usage.

2. Système d'analyse de lait selon la revendication 1, dans lequel les récipients à capteur (10) sont disposés sur une cassette mobile (8).

3. Système d'analyse de lait selon la revendication 23, dans lequel la cassette (8) comprend une bande souple (8a) sur laquelle sont formés les récipients à capteur (10) .

4. Système d'analyse de lait selon la revendication 3, comprenant un couteau (11) destiné à ouvrir chaque récipient (10), pour permettre au lait de passer par-dessus le capteur (9) respectif.

5. Système d'analyse de lait selon la revendication 4, comprenant une came (12) qui est utilisable pour déformer et reformer chaque récipient (10) afin d'appliquer un vide partiel dans le récipient ouvert (10), pour aspirer ainsi le lait par-dessus le capteur (9).

6. Système d'analyse de lait selon la revendication 4, comprenant :
une aiguille qui est utilisable pour perforer chaque récipient (10) ; et
une pompe qui est raccordée à l'aiguille et utilisable pour appliquer un vide partiel dans le récipient ouvert (10), pour aspirer ainsi le lait par-dessus le capteur (9).

7. Système d'analyse de lait selon la revendication 4, dans lequel chaque récipient (10) est configuré pour être préalablement mis sous vide, de telle sorte que l'ouverture du récipient (10) applique un vide partiel dans le récipient (10), pour aspirer ainsi le lait par-dessus le capteur (9).

8. Système d'analyse de lait selon une quelconque revendication précédente, comprenant un détecteur optique destiné à détecter des changements dans la couleur du capteur (9) à l'usage.

9. Système d'analyse de lait selon la revendication 8, comprenant un processeur configuré pour traiter une sortie du détecteur optique, pour fournir une indication immédiate, et/ou un enregistrement, du niveau du biomarqueur dans le lait.

10. Système d'analyse de lait selon une quelconque revendication précédente, le système étant disposé à l'intérieur d'un récipient étanche à l'eau, avec une entrée destinée à recevoir le lait et une sortie destinée à faire sortir le lait du système.

11. Système d'analyse de lait selon une quelconque revendication précédente, configuré pour communiquer avec un processeur distant destiné à traiter une sortie du système et fournir un rapport à un opérateur.

12. Procédé de fonctionnement d'un système d'analyse de lait selon l'une quelconque des revendications précédentes.

13. Procédé selon la revendication 12, dans lequel le biomarqueur en est au moins un parmi :
la progestérone en solution mesurée en nanogrammes par millilitre ;
l'œstradiol ;
l'hormone de stimulation folliculaire ;
une cétone ;
l'urée ;
une protéine de phase aiguë ;
une cellule somatique ; et
un anticorps contre un agent de maladie infectieuse.

14. Procédé selon la revendication 13, dans lequel le biomarqueur est un anticorps contre un agent de maladie infectieuse et la maladie infectieuse est la TB, la brucellose, ou la diarrhée virale bovine.
